# EUROPEAN PATENT APPLICATION

(11) **EP 1 643 246 A1**
(43) Date of publication of application: **05.04.2006**
(21) Application number: 04023456.9
(22) Date of filing: 01.10.2004
(51) Int. Cl.: G01N 33/36, G01N 21/898, D06H 3/08

(54) **Compact filamentous material detector**

(71) Applicant: Barco NV, 8500 Kortrijk (BE)
(72) Inventor: Deweer, Joris, 8510 Rollegem (Kortrijk) (BE)
(74) Representative: Bird, Ariane

(57) **Abstract**

The invention describes a system for detecting properties of elongate material such as textile material and a method for constructing such a system. The system comprises a light guiding device (230) wherein a measurement volume (304) is provided, at least one light source (212) and at least one light sensor (218). The light guiding device (230) exists of optical components that are shaped such that the light guiding device can be moulded in a single moulded piece. The lens (350) used to obtain a substantially parallel beam in the measurement volume (304) therefore has a specific convex shape. In a specific embodiment of the invention, the light source (212) and light sensor (218) are integrated on a single interconnection substrate (370) that is furthermore adjusted to position the light guiding means (230) at a calibrated position.

## Description

### Technical field of the invention

The present invention relates to a system and method for detecting and measuring characteristics of elongate materials such as wire or textile material and especially filamentous or tape materials such as natural and synthetic fibres, rovings, slivers, yarns, threads or similar materials. In particular, the invention relates to a system and method for optically measuring properties of such an elongate material, substantially independent of the exact position of the material in a detector measurement volume.

### Background of the invention

The quality of manufactured textile products depends strongly on the quality of the basic textile material used. A small unnoticed error in this basic textile material used for the manufacturing of a piece of textile or other textile related product, may lead to the piece of textile or textile related product being outside the quality requirements and thus being not sellable or at least being degraded in value. Detection of the properties of the basic textile material to guarantee its quality during manufacturing of textile products therefore is an important issue in the textile industry.

Different detection and monitoring systems to monitor the properties of elongate materials such as textile materials like natural and synthetic fibres, rovings, slivers, yarn, thread, etc. are known. The most common properties of elongate material monitored are the presence (i.e. whether or not an elongate material is broken), the thickness of an elongate material and the presence of foreign material in the elongate material, as the latter also often causes the resulted product to be defect. In some cases, also other elongate material properties such as colour, surface properties, chemical composition, transmissivity, absorbance, reflectance, etc. are monitored.

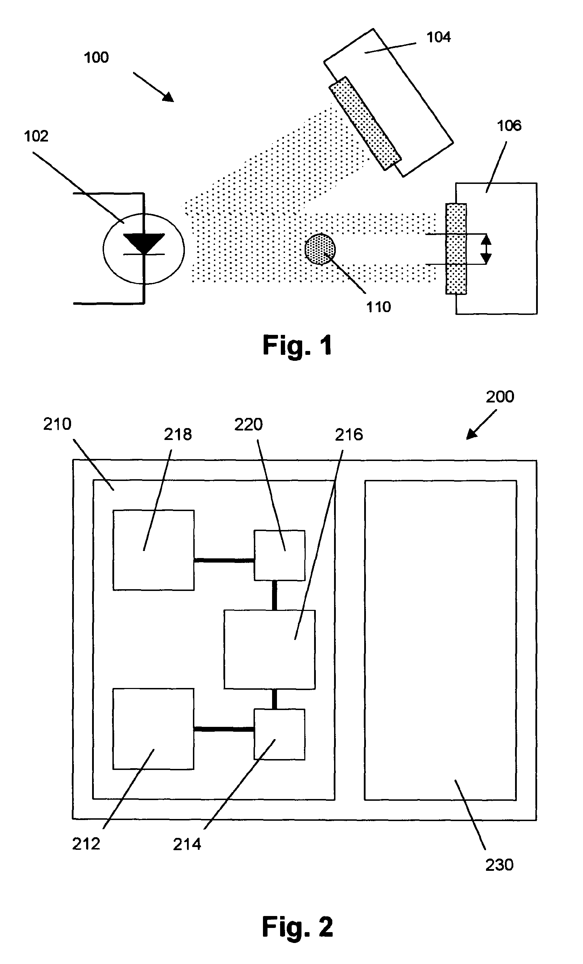

An illustration of a conventional system 100 for determining and/or monitoring the presence and the diameter of an elongate material is given in Fig. 1, wherein the general principle for a measurement based on shadow measurement is illustrated. A light source 102 emits radiation that is detected by two separate sensors 104, 106. A first sensor 104 measures the light intensity of the light source 102, by completely measuring the intensity of the light beam generated by the light source 102 and emitted in the direction of the first sensor 104, i.e. without influence of an elongate material such as filamentous or tape materials e.g. a natural or synthetic fibre, roving, yarn, thread, sliver, etc. In other words the measured signal is a reference signal representing the intensity of the light source 102. A second sensor 106 is positioned such that the light path of the light beam generated by the light source 102 in the direction of this second sensor 106 crosses the path of the elongate material 110, such as e.g. textile material. If the elongate material 110 is present, part of the light beam will be blocked and therefore part of the light beam will not be incident onto the second sensor 106. By comparing the signals received in both sensors 104, 106, the presence of the elongate material 110 can be detected. Furthermore, by evaluating the measured light intensity or by changing the second sensor 106 into a position sensitive sensor, a measure for the thickness of the elongate material 110 can be obtained. A disadvantage of this principle is that, without adjusted optics, the measured results are dependent of the exact position of the elongate material 110, as the shadow created will depend on the angle of incidence of the light rays.

The latter problem is at least partly solved by US 6,170,536. The application describes an apparatus for optically monitoring filling yarns. The invention relates to the detection of the presence and the position of a number of fillings. The system comprises a guide duct through which fillings pass. A light source creates light rays which are collimated substantially parallel in the guide duct by means of curved mirrors and a prism mounted in the housing. The light rays, possibly influenced by the presence of fillings, then are directed to an array of photodetectors to determine the presence and position of the fillings in the guide duct. The light source, the detector and the prism of the system described in US 6,170,536 need to be positioned in the housing of the apparatus for monitoring, which leads to time-consuming efforts and possible inaccuracies both during construction of the components, in order to manufacture the components within the specification of the housings, and during outlining of the system, in order to obtain a system that allows significant accurate measurement results. Furthermore a large number of components is used, which can drift during usage, leading to a decrease of accuracy in the measurement results over time.

It is a disadvantage of the systems known from the prior art, that they do not allow to obtain accurate measurement results that are not critically dependent on the position of the elongate material and whereby the detection and/or monitoring system has only a limited amount of different components that need to be manufactured and positioned accurately with respect to each other.

### Summary of the invention

It is an object of the present invention to provide a detection system and method allowing more accurate monitoring of properties of an elongate material such as a textile material or wire, substantially independent of the position of the material in the detector.

It is a further aspect of the present invention to provide a detection system and method requiring only a limited amount of components that need to be manufactured and positioned accurately with respect to each other.

The above objective is accomplished by a method and device according to the present invention.

In a first aspect, the present invention relates to an optical detection system for monitoring an elongate material in a measurement volume, the optical detection system comprising an optical detector with a light guiding device, said light guiding device being a moulded element comprising a means for imaging or conveying the illumination beam in said measurement volume, the means for imaging or conveying the illumination beam having at least one integrally moulded-in convex lens. The lens images the illumination beam into a bundle of parallel rays in the measurement volume.

Elongate materials to be monitored may be wire or textile material and especially filamentous or tape materials such as natural and synthetic fibres, rovings, slivers, yarns, threads or similar materials.

The measurement volume may be bound at one side by the convex lens or by a surface of the light guiding device adapted for guiding the illumination beam into the measurement volume, and at another side by a detection surface of an optical sensor or by a surface of the light guiding device adapted for guiding the illumination beam to such an optical sensor after the illumination beam has passed the measurement volume. The measurement volume thereby may be defined as the region between an optically emitting surface of the light guiding device, e.g. said convex lens, and an optically receiving surface, for receiving light after it has passed through the region. The measurement volume may furthermore be bound at one or more sides by other surfaces of the light guiding device or by a substrate, such as an interconnection substrate. In particular embodiments the measurement volume may be a slit, a hole or a recess in a light guiding device. The measurement volume is preferably limited to a region not larger than the region which can be illuminated by the formed beam of parallel light rays.

The moulded element may be a single moulded element. The single moulded element may be a single, integrally moulded element. The convex lens may have a curved shape having at least one centre of curvature. The centre of curvature of a curve at any point is defined as the centre of an osculating circle at that point. The illumination beam may pass outside each centre of curvature of said convex lens.

The means for imaging or conveying an illumination beam may comprise at least one reflecting surface to deviate the direction of said illumination beam.

The convex lens has a surface, wherein the surface may be a fractional barrel, a fractional cylindrical or fractional spherical surface or a truncated elliptical or a truncated oval surface. Fractional barrel may be half of a barrel or less, fractional cylindrical may be half of a cylinder or less and fractional spherical may be half of a sphere or less. The light guiding device may have a shape such that in a mould for moulding said light guiding device, the light guiding device has less than 5% undercut, preferably less than 1 % undercut, more preferably no undercut, even more preferably 1 % of overcut.

The illumination beam guided through the measurement volume may comprise radiation of a particular wavelength or radiation of a particular wavelength range and the moulded element may be made of material that is optically clear at said particular wavelength or in said particular wavelength range. Optically clear material thereby may be any of a plastic, a polycarbonate, an optically clear intrinsically antistatic material, a molded glass or a nylon.

The elongate material may be wire or textile material and especially filamentous or tape materials such as natural and synthetic fibres, rovings, slivers, yarns, threads or similar materials. The first optical detector may furthermore comprise at least one light source for generating the illumination beam and at least one optical sensor. The at least one light source, the at least one optical sensor and the light guiding device may be integrated on a single electronic interconnection substrate. The optical detector may allow measurement of a property of said elongate material, said property being at least one of diameter or dimension, presence, movement, colour, transmissivity, absorbance, reflectivity, a surface property, presence of a void or presence of a foreign material.

The at least one light source, the at least one optical sensor and the light guiding device may be positioned on calibrated positions of the electronic interconnection substrate. The electronic interconnection substrate may be a printed circuit board.

The first optical detector may furthermore comprise a second optical sensor for determining a reference signal from said light source.

The at least one optical sensor may be calibrated on said single electronic interconnection substrate so as to allow measurements in an absolute way.

The detection system may comprise at least one further detector having a sensor for measuring a property of said elongate material, whereby said property may be at least one of diameter, presence, movement, colour, transmissivity, absorbance, reflectivity, a surface property, presence of a void or presence of a foreign material. The sensor of the at least one further detector may be integrated on said single interconnection substrate.

The first optical detector and the at least one further detector may probe the elongate material in the same measurement volume. The convex lens may be adjusted such that measurement results are independent from movement of the elongate material both parallel to the light rays in the measurement volume and perpendicular to the light rays in the measurement volume.

The detection system furthermore may comprise a feedback system between said first optical detector and said at least one further detector.

The detection system may furthermore comprise a velocity detector for measuring the speed of a moving material, said velocity detector being integrated on said single interconnection substrate. The convex lens may be shaped such that said property can be determined substantially independent of the exact position of the elongate material in the measurement volume.

In the optical detection system, the at least one light source may be stabilised electronically. The at least one light source may comprise a group of light sources.

In the optical detection system, the first optical sensor may be a segmented optical sensor. Part of the segmented optical sensor may be used to obtain a reference signal from said at least one light source. On the other hand, the first optical sensor also may be a detector array.

A protection means may be provided to seal the detection system from dust and/or scratches or to shield for electrostatic, magnetic and/or electromagnetic fields. The protection means may comprise glass plates in the measurement volume. The measurement volume may be provided with a means for making the measurement volume conductive and/or anti-static.

The invention furthermore relates to a second aspect, i.e. a method of manufacturing a detection system for monitoring elongate material, the method comprising providing an electronic interconnection substrate, integrating a light source and at least one light detector on said interconnection substrate, and positioning an integrally moulded piece onto the interconnection substrate to obtain optical components for the detection system. Elongate materials may be wire or textile material and especially filamentous or tape materials such as natural and synthetic fibres, rovings, slivers, yarns, threads or similar materials. The method of manufacturing may furthermore comprise providing protection means for sealing said detection system from dust or for shielding for electrostatic, magnetic and/or electromagnetic fields.

The present invention furthermore relates to a light guiding device for guiding an illumination beam in a region adapted for receiving elongate material to be measured, said light guiding device being a moulded element comprising a means for imaging or conveying the illumination beam in said region, the means for imaging or conveying comprising at least one integrally moulded-in convex lens.

The light guiding device may comprise any of the features of the light guiding device described above as component of an optical detection system. It is an advantage of the present invention that the detection and/or monitoring system can be manufactured with a reduced amount of effort. It is furthermore an advantage of the present invention that the accuracy of the detection and/or monitoring system is less dependent on drift during usage and less dependent on the outlining of the different components during manufacturing.

It is an advantage of the present invention that all electronic components can be integrated on a single interconnection substrate, therefor allowing that the detection system is substantially flat and small in size.

It is an advantage of the present invention that the optical components of the detection system can all be manufactured together in a single moulded piece. It is furthermore an advantage of the present invention that the single moulded piece can be made using one single mould, thereby avoiding seams or break lines over the piece, especially over the optical components such as lenses and mirrors.

It is an advantage of the present invention that the detection system can be sealed from dust.

It is an advantage that the optical components can be decoupled easily from the electronic components.

It is an advantage of the present invention that the detection system can be used for optically measuring a variety of properties of an elongate material such as e.g. textile material or wire, such as the presence of the elongate material, the thickness or diameter of the elongate material, the reflectivity, the absorbance and the transmissivity of the elongate material, the colour of the elongate material, the presence of voids in the elongate material, the presence of foreign fibres in the elongate material, or surface properties of the elongate material, e.g. the presence of hairs, etc.

Although there has been constant improvement, change and evolution of devices in this field, the present concepts are believed to represent substantial new and novel improvements, including departures from prior practices, resulting in the provision of more efficient and reliable devices of this nature.

The teachings of the present invention permit the design of improved methods and apparatus for detection and/or monitoring of properties of elongate materials such as textile material or wire.

These and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference numbers quoted in the text below refer to the attached drawings.

### Brief description of the drawings

Fig. 1 shows a schematic representation of a presence and/or thickness of elongate material measurement system implementing a shadow measurement, as known from the prior art.
Fig. 2 shows a schematic block diagram of a detection system for detecting and monitoring elongate material according to embodiments of the present invention.
Fig. 3 is a schematic representation of a surface properties optical sensor that can be used for monitoring surface properties according to a first embodiment of the present invention.
Fig. 4 is an x-z cross-sectional representation of a detection and/or monitoring system according to the first embodiment of the present invention.
Fig. 5 is an elevated bottom view of a light guiding structure for a detection and/or monitoring system according to the first embodiment of the present invention.
Fig. 6 is a schematic representation of a fractional spherical lens which can be used in a detection and/or monitoring system according to any of the embodiments according to the present invention.
Fig. 7 is a schematic representation of a detection and/or monitoring system according to a second embodiment of the present invention.
Fig. 8 is a schematic representation of a light guiding structure according to a third embodiment of the present invention.
Fig. 9 is a schematic representation of measurement conditions for testing the position independence of the measurement results obtained with detection and/or monitoring systems according to embodiments of the present invention.
Fig. 10 is a graphical representation of the test results for position independence of the measurement results obtained with a detection and/or monitoring system according to embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

In the following the present invention will be described with reference to elongate material, such as e.g. textile material and especially filamentous or tape materials such as natural and synthetic fibres, rovings, slivers, yarns, threads or similar materials. Other types of materials such as wires also can be monitored. Although the present invention will mainly be described with respect to moving elongate materials, movement of the object under test is not a requirement of the present invention. Objects may be placed in a detection zone of a detector according to the present invention, e.g. on a stationary table.

In a first embodiment of the present invention, a detection system for monitoring properties of an elongate material, such as e.g. twisted, knitted or woven materials or threads, yarns, strings, braids, ropes, rovings or slivers or similar materials is described. The detection system can be used both for moving elongate material especially textile material, such as e.g. a moving yarn during textile related processing, or for a stationary elongate material, especially textile material. The detection system claimed measures and/or monitors properties of elongate materials using an optical detection technique. Several properties can be measured using an optical technique: the thickness of e.g. an elongate material, the presence, the transmissivity, the reflectivity, the absorbance, the colour, the presence of voids, the size of voids, the surface properties such as hairs that are present at the surface, the presence of foreign materials, etc in any suitable material.

The detection system 200 according to the first embodiment comprises electronic components 210 and their interconnections on the one hand and optical components 230 on the other hand, which are schematically shown in Fig. 2. The electronic components 210 typically comprise at least one light source 212 with a driving means 214 for the light source 212 powered by a power supply 216 and at least one optical sensor 218, driven by a driving means 220 powered by the power supply 216. The power supply 216 for different electronic components may be the same for a plurality of electronic components, or each electronic component or group of electronic components may have its own power supply. The optical components 230 typically comprise a first optical guiding means (not shown in Fig. 2) for guiding an illumination beam from the at least one light source 212 to a measurement volume wherein interaction of the illumination beam with the elongate material under test, e.g. a textile material, can occur and a second optional optical guiding means (not shown in Fig. 2) for guiding the illumination beam, after interaction with the elongate material, further towards the at least one optical sensor 218. The illumination beam transmitted by the optical components 230 is adjusted with a lens (not shown in Fig. 2) to image the illumination beam into a bundle of parallel rays over the measurement volume. This allows to perform detection and/or monitoring of elongate material substantially independently of the exact position of the elongate material in the measurement volume. It is a specific advantage of the present invention that the different components or sets of components of the optical components 230 are adjusted so that the optical components 230 can be manufactured all together in a single moulded element. This allows more accurate measurements because the different optical components 230 are located in a precise relationship to each other and cannot shift relative to each other, as drift of the different components relative to each other during use of the detection system is prevented. Furthermore time-consuming calibration procedures for positioning the different optical components relative to each other can be avoided. As a set of the optical components 230 is made in one single element, also the manufacturing effort is reduced. The different components of the detection system 200 as shown in Fig. 2 will now be described in more detail.

The at least one light source 212 used depends on the type of property that is to be measured with the optical sensor 218. The wavelength or wavelength range of the at least one light source 212 can have any suitable value, e.g. in the ultraviolet part of the spectrum, the infrared part, the far-infrared part or the visual part of the electromagnetic spectrum, depending on the application. The at least one light source 212 also can be a combination of several light sources 212, allowing combination of specific wavelengths or wavelength ranges of emission, depending on the application of use. In this case the light source 212 can be e.g. a combination of light emitting elements such as diodes each emitting radiation of specific wavelengths or wavelength ranges. Also various light sources may be provided which need not all to be used at the same time. For e.g. measurement of the thickness or the presence of an elongate material, the at least one light source 212 used may be any light source that generates light in a wavelength range that is sufficiently absorbed or reflected by the elongate material. In this way, the contrast on the optical sensor 218 for light rays that are not influenced and light rays that are influenced by the material is sufficiently distinct. Typically the wavelength range used in these applications is infrared light, because of the absorbance characteristics of infrared light by most elongate materials such as textile materials. The absorbance or reflectance requirements as described above are also important if e.g. properties such as presence of voids and surface properties are studied based on shadow measurements. In case the colour of the elongate material is to be determined, the light source 212 typically will be a white light source comprising light of all wavelengths in the visual light range. Also for measurements of reflectivity, transmissivity and/or absorbance characteristics, a light source 212 having a broad range of wavelengths can be used. For the detection of foreign material in elongate material, often the different reflectivity, transmissivity and/or absorbance characteristics of the foreign material compared to the elongate material are used. This can either be checked for light of specific different wavelengths or this can be checked over a more broad wavelength range. Furthermore also fluorescence measurements could be performed to check foreign material, in which case the light source 212 is adapted to create fluorescence.

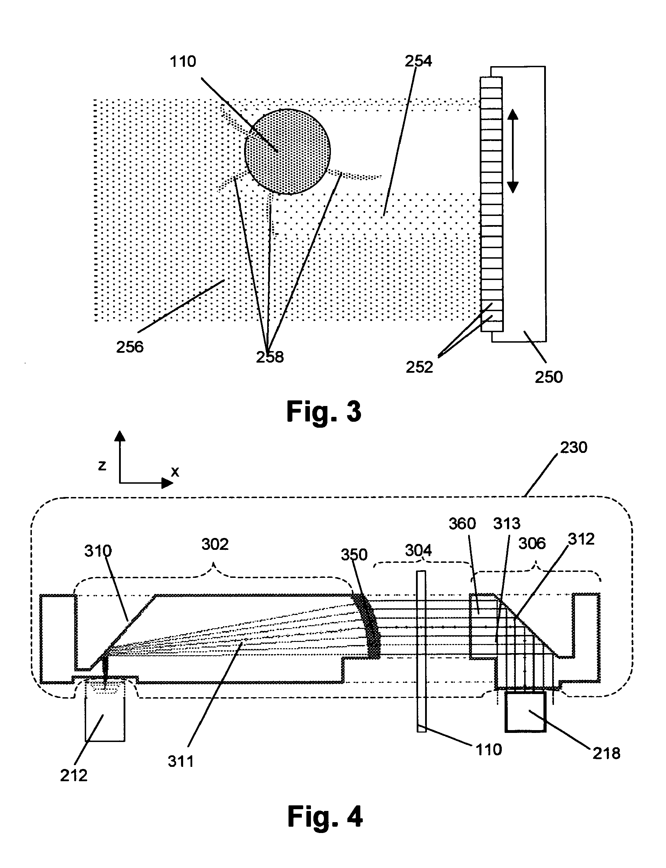

The at least one optical sensor 218 used, depends on the property of the elongate material under test. If detection of the presence of elongate material or monitoring of the thickness of elongate material is needed, typically a single element optical sensor 218 can be used, although the invention is not limited thereto, whereby the amount of detected light is a signal of the presence (or absence) of elongate material or the amount of detected light is a measure for the diameter of the elongate material. If the absorbance or reflectivity by the elongate material is large enough, i.e. if the transmissivity through the elongate material can be neglected, no further information is needed to obtain a measure for the diameter of the elongate material. If the transmissivity is not neglectable, the transmissivity properties for that type of elongate material need to be known for the wavelengths of the light source 212 used, in order to obtain a good measure of the diameter of the elongate material. The optical sensor 218 needs to be chosen in agreement with the at least one light source 212 used, i.e. the optical sensor 218 should be sensitive for the wavelengths emitted by the light source 212 that is used. If e.g. the surface properties of the detection system are studied, the optical sensor 218 used typically is an array optical sensor 250 which may comprise a number of pixels 252. An example of an optical sensor arrangement for measurement of the surface properties of an elongate material is schematically illustrated in Fig. 3. The optical sensor principle that is used for this application is based on a shadow measurement, i.e. an illumination beam that is first directed onto the elongate material to be measured, is subsequently guided to the optical sensor 250. Parts 254 of the illumination beam that are influenced by elements of interest 258, e.g. hairs or roughness on the surface of the elongate material 110, have a light intensity that is substantially different from the light intensity absorbed by the bulk elongate material 110, as the hairs 258 on the surface of the elongate material will have absorbed or reflected the light differently than the bulk elongate material 110. Parts 256 of the illumination beam that are not influenced by the elongate material 110, 258 have a light intensity that is not changed compared to the original light intensity. The pixel size of pixels 252 may be adjusted so that the elements of interest 258 such as hairs on the surface of an elongate material 110 can be measured with a significant accuracy. The optical sensor array 250 may be a line optical sensor or, to improve the accuracy and the amount of obtained information, a matrix optical sensor. It will be obvious for the person skilled in the art that an optical sensor array 250 can also be used to measure the thickness of the material or to check the presence of elongate material 110. If wavelength dependency of properties is essential, e.g. for absorbance, transmissivity, reflectivity or during checking of the presence of foreign material, the optical sensor 218 can be adjusted to be sensitive for these wavelengths, e.g. by the use of filters. Furthermore if the wavelength dependency in a broader wavelength range needs to be studied, the optical sensor 218 typically can be a spectrophotometer, allowing wavelength dependent detection and/or monitoring.

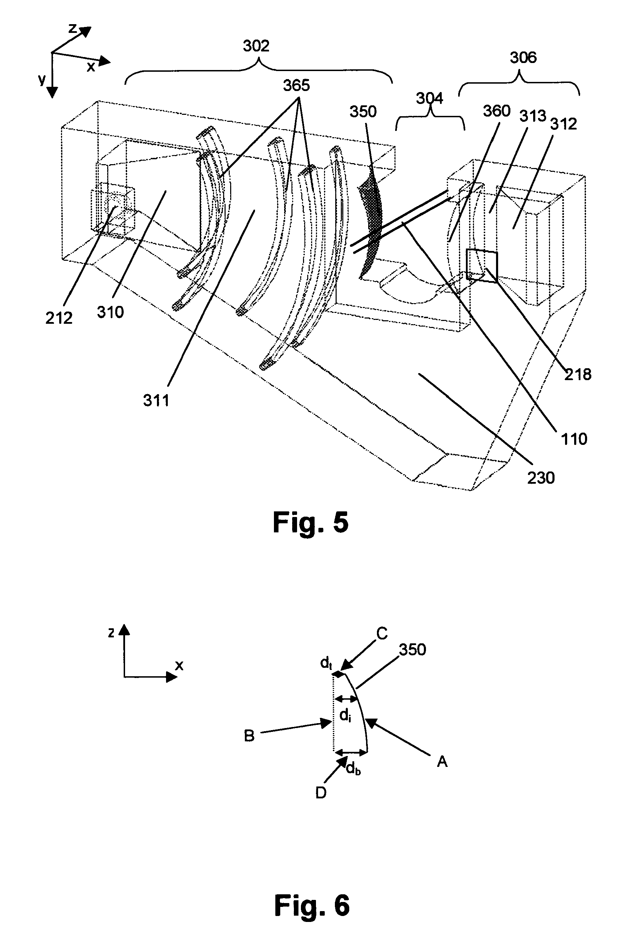

The optical components 230 of the detection system 200, as illustrated in Fig. 4 and Fig. 5, allow guidance of the illumination beam of the light source onto a measurement volume in which an elongate material to be tested, e.g. a textile material, can be positioned. These optical components 230 comprise first optical guiding means 302 for guiding the illumination beam from the light source 212 to a measurement volume 304, wherein the illumination beam can interact with an elongate material 110 under test e.g. a textile material. After passing the measurement volume 304, and thus after the possible interaction with the elongate material 110 under test, e.g. the textile material, the illumination beam is guided towards the at least one optical sensor 218, e.g. by an optional second guiding means 306. Both first guiding means 302 and second guiding means 306 for guiding the light may comprise reflecting surfaces 310, 312 such as mirrors or gratings to change the direction of the illumination beam. In an alternative design (not represented in the drawings), the light source 212 and the optical sensor 218 may be positioned in line with each other, the measurement volume 304 being located in between the light source 212 and the optical sensor 218. The illumination beam then does not need to be redirected by reflecting surfaces 310, 312 such as mirrors or gratings.

The first and second light guiding means may each comprise a light channel 311 and 313 in which the illumination beam is propagated.

The optical components 230 include at least one first imaging means which is a lens 350 for guiding the illumination beam in the measurement volume 304 such that the illumination beam in the measurement volume 304 consists of parallel light rays.. In order to optimally provide parallel light rays in the measurement volume 304, the lens 350 has a surface with a convex shape, for example a curved shape, like e.g. a surface having a shape being a fractional cylindrical surface, a fractional barrel surface, a fractional parabolic surface, a fractional elliptical surface, a fractional oval or a fractional spherical surface, i.e. the side of the lens oriented to the measurement volume 304 has a shape which is a fraction of a cylindrical, barrel, parabolic, oval, elliptical or spherical shape. The fraction is preferably one half or less. The fractional spherical shape allows that the illumination beam which is slightly diverging before it is incident on the lens, is converted in a parallel beam of light rays. The fact that only a fraction of a spherical shape is used, especially when the fraction is less than one half, can allow manufacture of at least some, and preferably all, of the optical components 230 in a single moulded element.

The illumination beam furthermore has a significant width, which allows detection or monitoring of properties of an elongate material 110 under test, e.g. a textile material, that might be present in the measurement volume 304 over a quite broad depth of the measurement volume 304. In other words, by creating the broad parallel illumination beam in the measurement volume 304, the exact position of the elongate material 110 under test, e.g. the textile material, does not significantly influence the measurement anymore. The amount of allowed deviation of the elongate material 110 under test from the central position of the optical axis is determined by the convex lens 350 used and by the specific set-up of the light source 212 and the optical components. This may be e.g. a deviation of up to 20% of the aperture of the first optical guiding means 302 at both sides of the central position in Y-direction. The system also provides an independency of the exact position of the elongate material 110 with respect to the direction parallel to the light rays incident on the measurement volume 304. This may be e.g. deviation of up to 15% of the aperture of the first optical guiding means at both sides of the central position in X-direction. The measurement thus is substantially independent of the exact position of the elongate material 110 in the measurement volume 304. The advantage of the detection and/or monitoring of the properties of the elongate material 110 being independent of the exact position of the elongate material 110 is typically not obtained in conventional systems not having a convex lens 350 such as e.g. fraction of a spherical lens. In systems wherein no parallel beam is created in the measurement volume 304, the detection and/or monitoring system would either be not able to detect and/or monitor an elongate material 110, i.e. if the elongate material 110 is deviated too far from the central position, or would give an erroneous result, i.e. the shadow of the elongate material 110 or of the corresponding hair 258 will be projected under an angle corresponding with the angle of incidence of the light rays of the illumination beam.

After passing the measurement volume 304, the illumination beam is either guided directly onto detector 218, such as e.g. the position dependent detector discussed above, or the illumination beam is guided to the optical sensor 218 by means of a light guiding means 306. The light guiding means 306 therefore may comprise a second imaging means 360 to image the illumination beam onto the optical sensor 218. The second imaging means 360 may be a lens having a specific shape at the side oriented to the measurement volume 304. The lens 360 may have a surface having any suitable shape such as a convex shape, e.g. a barrel shape, a cylindrical shape, an elliptical shape, a parabolic shape, a spherical shape or a fraction thereof. To ease manufacture and release from a mould, the lens 360 preferably has a surface having a fractional barrel shape, a fractional cylindrical shape or a fractional spherical shape, the fraction preferably being one half or less, as this allows the second imaging means to be integrally moulded in into the light guiding device along with at least some of the other optical components 230.

The shape of the lens 350, the second imaging means 360 and the optical guiding means 302, 306 thus are such that the optical components can be moulded in a single element. This not only leads to the advantage that manufacturing of these components is substantially easier, it also allows to avoid positioning problems of the different optical components with respect to each other, which allows to obtain more accurate measurement results, avoids time consuming calibration procedures for positioning the different components and drift between the different optical components during use. Avoiding drift significantly improves the accuracy of the measurement results. During manufacturing the single moulded element can be made using a single, unitary or integral mould. The single moulded element of optical components 230 may be made of an optically clear material such as e.g. acrylonitrile butadiene styrene (ABS), polycarbonate, polyethylene, polypropylene, high density polyethylene, polyamide nylon, plastic, optically clear intrinsically antistatic material, molded glass etc. In order to remove the optical components from the single mould, the amount of undercut should be limited or even no undercut should be present, depending on the elasticity of the material and/or of the mould used. The undercut should be limited to less than 5%, preferably less than 1 % if the elasticity of the moulded piece or the elasticity of the mould is significantly large so the piece can be removed from the mould without damaging it. If the material from which the optical components are made or the material of the mould is not very elastic, no undercut should be present. Depending on the elasticity of the moulded piece material and/or the mould material, it can even be necessary to provide at least a small amount of overcut, such as e.g. 1 %.

An example of an x-z cross-section of a convex lens 350 that can be used in single moulded optical elements according to the present invention is shown in Fig. 6, based on its positioning in the x-z cross-section shown in Fig. 4. The convex lens 350 on one side A has a convex shape which preferably is a fraction of a spherical shape, barrel shape, cylindrical shape, ellipsoidal shape, parabolic shape etc.. On the opposite side B, the lens may be either substantially flat, have another shape or may converge in the light channel 311 of the first light guiding means 302. The convex lens 350 thereby is such that it provides no undercut in a mould. In other words, the convex shape at the side A of the convex lens 350 is chosen such that, with reference to the z-direction in the x-z diagram in Fig. 4 and Fig. 6, the diameter dₜ at the top side C of the lens 350 is smaller than the diameter d_{b} at the bottom side D of the lens 350. Furthermore, the fraction is chosen such that for every intermediate diameter dᵢ**,** the diameter is smaller than all diameters for parts lower in the lens, i.e. for parts closer to side D. In this way, a single mould can be removed in the direction of the z-axis as indicated in Fig. 4 and Fig. 6, i.e. from the bottom side D to the top side C of the lens 350. Alternatively, the convex lens 350 may be such that the diameter at the top of the lens 350 is larger than the diameter at the bottom of the lens and that every intermediate diameter is larger than for parts lower in the convex lens 350. The mould then is to be removed in the negative z-direction and the convex lens 350 then is present in another part of the mould than the other optical components.

The lenses, mirrors and the light imaging means of the different light guiding means may be covered with a layer of material, e.g. an antireflection coating, an antifogging coating, a coating having a substantially different refractive index, to improve the optical properties of these optical components. During manufacturing, this layer may be provided on pre-shaped optical components and these components may be subsequently co-injected in the single mould.

The first light guiding means 302 may furthermore comprise further means 365 for reducing the amount of stray light. These further means 365 may be provided by limiting the thickness of the light guiding means 302 through which the illumination beam is guided from the reflecting surface 310 to the convex lens 350, i.e. for example by providing grooves at the top and the bottom of the light guiding means 302. These grooves furthermore allow an improved control of the shrink of the moulded element during manufacturing. The optional further means 365 are shown in Fig. 5. The grooves can optionally be filled with light absorbing material.

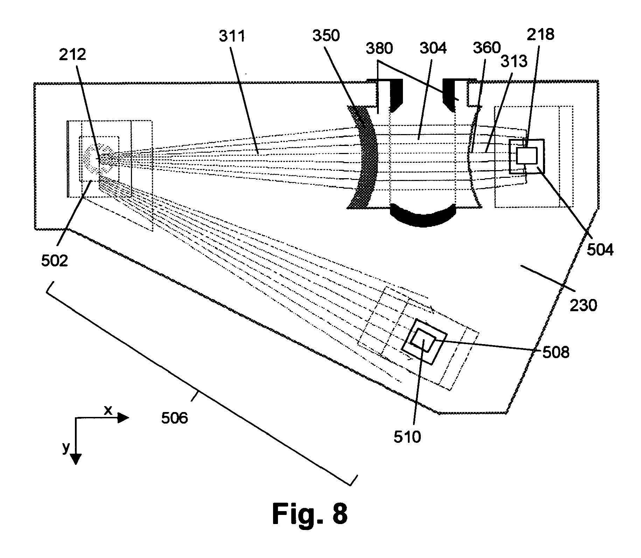

The detection system furthermore may be provided with means for sealing (not shown in Fig. 4 and Fig. 5) the optical components 230 and the electronic components 210 from dust. These sealing means may comprise glass plates, shifted in the measurement volume 304, but are not limited thereto. Other materials such as scratch hardened plastics, e.g. SiO₂ coated plastics, also can be used. This not only allows sealing of the components of the detection system from dust, but it also provides the possibility for cleaning the system without damaging e.g. the optical components 230. The means for sealing 380 are illustrated for a further embodiment in Fig. 8. These means for sealing 380 furthermore may be adjusted such that the measurement volume 304 is antistatic or conductive.

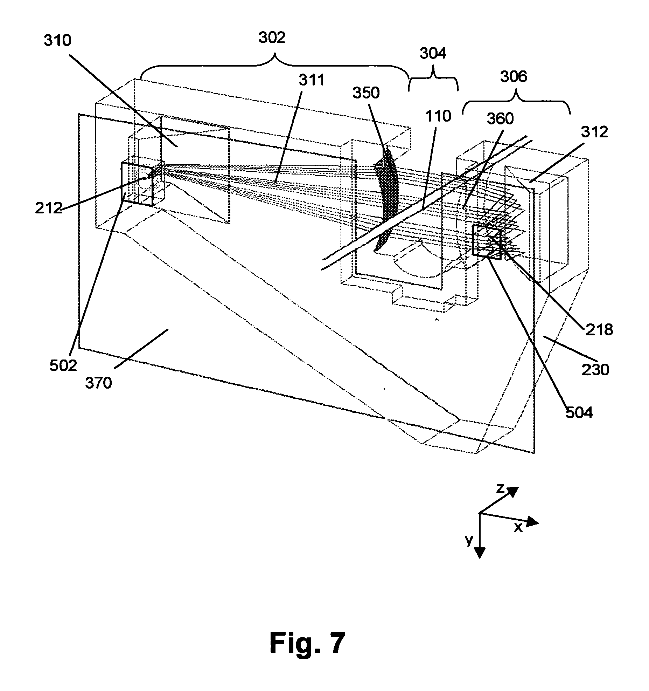

In a second embodiment, the detection system comprises the components and features of the above described embodiment wherein the optical components 230, grouped in a single moulded element and further referred to as the light guiding structure 230, receive the illumination beam at one side, e.g. at the bottom, of the light guiding structure 230, from at least one light source 212 and the at least one sensor 218 both incorporated on common an interconnection substrate 370. The detection system according to the present embodiment is shown in Fig. 7. The bottom of the light guiding structure 230 thereby is defined by that side of the light guiding structure 230 which corresponds with the smallest z-values, the z-axis being as indicated in Fig. 7. The light source 212 on the interconnection substrate 370 emits an illumination beam from the bottom side of the light guiding structure 230 in the z-direction. The light guiding structure 230 is adjusted to receive the illumination beam through entrance opening 502. The first light guiding means 302 directs the illumination beam from the light source 212 towards the measurement volume 304. The first light guiding means 302 comprises a reflecting surface 310 to direct the illumination beam from the z-direction substantially into the x-direction. The reflecting surface 310 typically is put under an angle such that a good spreading of the illumination beam is obtained at the surface of the convex lens 350. The illumination beam thus diverges towards the convex lens 350, which collimates the illumination beam into a bundle of parallel light rays. In this way, a large part of the measurement volume 304 can be illuminated. The convex lens 350 may be e.g. any of a fractional barrel lens, a fractional cylindrical lens, a fractional parabolic or a fractional spherical lens, with the fraction preferably being one half or below as described in the previous embodiment. If elongate material 110 is present in the measurement volume 304, the bundle of parallel light rays interacts with the elongate material 110. If no elongate material 110 is present, the bundle of parallel light rays is not influenced. After the illumination beam has passed the measurement volume 304, the illumination beam is directed by a reflective surface 312 comprised in a second light guiding means 306 through light output position 504 towards the light sensor 218. The light sensor 218 is positioned at the bottom of the light guiding structure 230 on the common interconnection substrate 370. The present embodiment therefore allows to obtain an illumination beam in the z-direction from a light source 212 and to emit the illumination beam in the z-direction towards the light sensor 218. The second light guiding means 306 may comprise a second imaging means 360 which may be a convex lens such as e.g. a fractional spherical lens, a fractional parabolic lens, a fractional cylindrical lens or a fractional barrel lens, especially a fractional lens with the fraction being one half or below as described in the first embodiment.

The specific set-up of the light guiding structure 230 of the second embodiment of the invention allows that in the present invention the electronic components are integrated on a single interconnection substrate 370. This allows that the detecting system is flat and small in size which is an important advantage as the detection system very often needs to be integrated in textile or other processing machinery where the available amount of space is limited. The interconnection substrate 370 can be an electronic interconnection substrate such as e.g. a printed circuit board, but it is not limited thereto. The interconnection substrate 370 also may be e.g. a flexible substrate which allows interconnecting integrated components. In other words, in this embodiment all electronic components can be grouped on a single interconnection substrate 370 thereby reducing the size of the detection system. Furthermore, the amount of shift between the different electronic components is reduced or even completely deleted as the electronic components may be completely integrated on the interconnection substrate 370. This allows to make the system more compact. The use of a single moulded element which groups the optical components allows to reduce possible shifts between the electronic components and the optical components, or between the optical components themselves. The use of grouped electronic components integrated on a single interconnection substrate 370 is not limited to be combined with the optical components as described in the above embodiments. In other words, in general, all optical components that allow to guide an illumination beam from the light source to a measurement opening and that allow to guide the radiation, resulting from the interaction between the illumination beam and the material to be measured, to the sensor, can be used.

Furthermore, the interconnection substrate 370 may comprise the processing means for processing the signals obtained using the sensors 218. Such a processing means (not shown) may be dedicated circuitry such as e.g. a microcomputer, a digital signal processor (DSP), a general purpose processor, an application specific integrated circuit (ASIC), a microprocessor, etc. The interconnection substrate 370 may be adjusted such that the light guiding structure 230 can be, during manufacturing, positioned on a calibrated position on the interconnection substrate 370. This may e.g. be done totally or partly by automatic mounting machines such as e.g. standard precision automatic mounting machines, which allows to mount additional compatible electronic or other components on the same interconnection substrate 370. These additional compatible electronic components may form part of the detection system circuitry or part of another electronic circuit.

In a third embodiment, the detection system as described in the first and/or the second embodiment furthermore comprises a second light sensor. A schematic illustration of the light guiding structure 230 for such a detection system is shown in Fig. 8. Light from the light source which enters the light guiding structure 230 through entrance opening 502 is directed by a third light guiding means 506 to a second light output position 508, where a second light sensor (510) can be positioned. The second light sensor (510) obtains the light directly from the light source, i.e. without being influenced by the presence of an elongate material (not represented in Fig. 8) such as e.g. a textile material. This signal can be used as a reference signal, so that intensity fluctuations of the light source do not influence the results of the measured properties of the elongate material measured. In other words, the illumination beam then is split in two different illumination beams, i.e. an illumination beam which will be used as a reference beam and an illumination beam which will be used for the measurement of a property of an elongate material such as a textile material which may be e.g. a yarn, sliver, thread, fibre, etc.

In still an alternative embodiment of the present invention, the reference signal may be obtained by providing a single segmented light detector, such that the reference signal can be measured on one of the segments of the detector which is not influenced by the elongate material. Another alternative may be providing a stable light source due to an accurate electronic driving of the light source, thereby allowing to avoid the need for measuring a reference signal.

In a fourth embodiment, the detection system as described in one of the previous embodiments, i.e. comprising the same components, features and set-up as any one of these previous embodiments, furthermore comprises a second detector, which if this is an optical detector can use the same light source 212 or use a different light source and which uses a separate optical sensor so that either the at least one property measured with the first optical detector is measured as a control measurement or at least one other property can be measured. The second detector thus allows measurement of at least one of the following properties of the elongate material, i.e. thickness, presence, transmissivity, reflectivity, absorbance, colour, presence of voids, size of voids, surface properties such as hairs that are present at the surface, presence of foreign materials, etc. The second detector preferably is such that the optical components used to guide the light in the second optical detector also are integrated in the single moulded element just like the optical components of the first optical detector. Alternatively, the second detector can also be a velocity detector. Velocity detectors are known by the person skilled in the art.

Furthermore, it is advantageous if the electronic components are also integrated on the interconnection substrate 370.

The detection system may furthermore comprise a feedback system which provides information between the two optical detectors. This allows to obtain more accurate results, resulting in an improved monitoring/control of the elongate material. A typical combination of properties that are measured are the thickness of the elongate material such as e.g. the textile material and the presence of foreign material.

In a fifth embodiment, the detection system as described in any of the previous embodiments may also comprise an output system (not represented in the drawings) that allows providing output information about the measured properties of the elongate material 110 such as e.g. textile material to an external control system. In this way the detection system may allow to provide feedback to the appliance using the elongate material. If the properties of the elongate material 110 are not within certain boundary conditions, a signal may be sent to a control system of an appliance allowing to stop the appliance and thus preventing the use of e.g. low quality elongate material 110 in the appliance. Deviating properties are measured if the elongate material 110 is not present i.e. if for example a yarn has broken, or if too much foreign material is present, or if the diameter is outside certain specifications.

According to a further aspect, the invention furthermore relates to any of the light guiding structures for guiding light from a light entrance position 502 to a measurement volume 304 and further from the measurement volume 304 to a light output position 504 as described in the previous embodiments. It is a specific advantage of the light guiding structures according to embodiments of the present invention that they can be moulded as a single element in a single mould. The shape of all optical components present in the light guiding structure therefore is such that it allows the use of a single mould to manufacture the single moulded element, avoiding the presence of break lines, which are present when several pieces are fixed together whereby moulding in a single mould is not possible. In order to obtain this, the shape of the optical components is such that in the corresponding mould of the light guiding piece, the amount of undercut is strongly limited or that even no undercut is present. Depending on the elasticity of the material used for the optical components or for the mould used for moulding, the undercut should be reduced to smaller than 5%, preferably smaller than 1 %, more preferably no undercut should be present. Further depending on the elasticity of the materials used, it could even be advantageous to have a small overcut, so that during removal of the piece out of the mould, the surface of the optical components is not damaged. Adjusting of the shapes of the optical components is done as described in the previous embodiments, i.e. for example by limiting the lenses used to convex lenses having e.g. only a fractional spherical, barrel, parabolic, elliptic, cylindrical lens whereby the fraction is half or less then half. Typical materials used for the light guiding structure are optically clear materials, such as e.g. acrylonitrile butadiene styrene (ABS), polycarbonate, polyethylene, polypropylene, high density polyethylene, polyamide nylon, plastic, moulded glass, optically clear intrinsically antistatic material, etc.

The first and, if present, the third light guiding means 302, 506 furthermore may comprise further means 365 for limiting the amount of stray light. These further means 365, may be provided by e.g. limiting the thickness of the light channel 311 in the first light guiding means 302 through which the illumination beam is guided, i.e. by providing grooves in the light guiding structure 230. The reflective surfaces and the light imaging means of the different light guiding means 302, 306, 506, may furthermore be covered, e.g. with an antireflection coating, an antifogging coating or with a layer of high refractive index, to improve the optical properties of these optical components. This layer may be provided first and the optical components then may be co-injected in the single mould.

In all the above described embodiments, the light passes the imaging means in an off-centre position. In other words, the illumination beam in the light guiding structure does not pass through the centre of curvature of the convex lens.

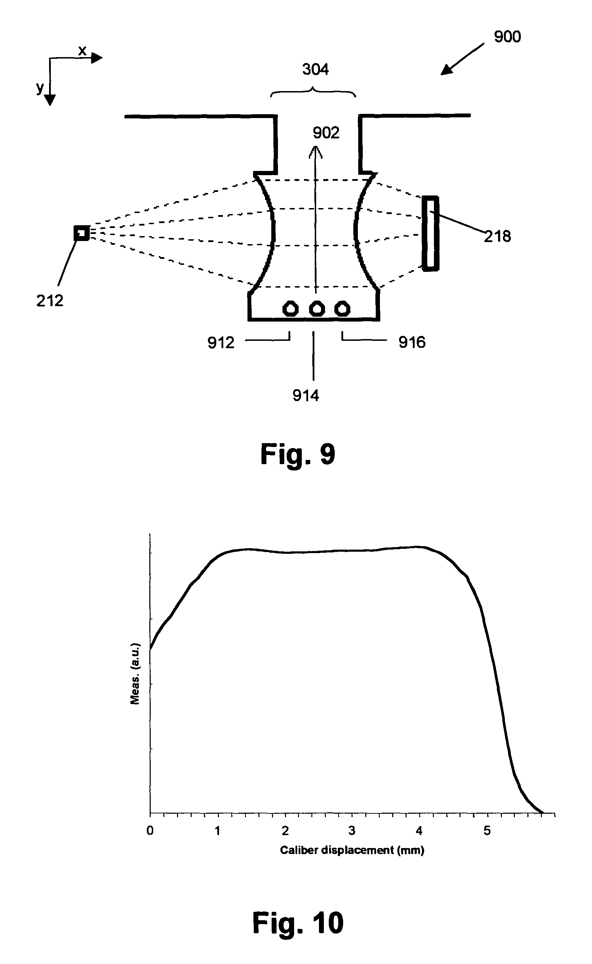

A detection system according to the above described embodiments of the present invention has been checked for accuracy and position independence of the elongate material. In this example the diameter of the material is monitored. First a calibration material is inserted in the centre of the measurement volume 304 and the light guiding structure 230 is positioned optimally with respect to the interconnection substrate 370 such that the largest independence is obtained for shift of the calibration material in the direction perpendicular to the light rays crossing the measurement volume 304, i.e. in the direction of the arrow 902 in Fig. 9. By way of example, an illustration is given for a measurement volume of 5 mm (x-direction) by 6 mm (y-direction). A shift of up to about 4 mm then still allows detection and accurate measurement of the thickness of the calibration material. Furthermore, if the calibration material is shifted in the direction parallel to the light rays crossing the measurement volume, i.e. for calibration material positions 912, 914, 916 between the convex lens 350, 360, the same detection accuracy can be obtained over a width of about 4 mm. So both in the direction parallel to the light rays crossing the measurement volume 304 and in the direction perpendicular to the direction of the light rays crossing the measurement volume 304, the system can provide accurate measurements of the diameter of the material. The accuracy with which the diameter could be determined is about 5 µm. The corresponding results are shown in Fig. 9, indicating the diameter measurement result as a function of a shift perpendicular to the light rays, i.e. in the direction of arrow 902.

Other arrangements for accomplishing the objectives of the method and system for detecting and monitoring properties of elongate material embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

## Claims

1. An optical detection system for monitoring an elongate material (110) in a measurement volume (304), the optical detection system comprising a first optical detector with a light guiding device (230), wherein said light guiding device (230) is a moulded element comprising a means for imaging or conveying the illumination beam in said measurement volume, the means for imaging or conveying comprising at least one integrally moulded-in convex lens (350).

2. An optical detection system according to claim 1, the convex lens (350) having a curved shape having at least one centre of curvature, wherein said illumination beam passes outside each centre of curvature of said convex lens (350).

3. An optical detection system according to any of claims 1 or 2, wherein said means for imaging or conveying an illumination beam furthermore comprises at least one reflecting surface (310) to deviate the direction of said illumination beam.

4. An optical detection system according to any of claims 1 to 3, said convex lens (350) having a surface, wherein said surface is a fractional barrel, a fractional cylindrical or fractional spherical surface.

5. An optical detection system according to any of claims 1 to 4, said illumination beam comprising radiation of a particular wavelength or radiation of a particular wavelength range, wherein said moulded element is made of material that is optically clear at said particular wavelength or in said particular wavelength range.

6. An optical detection system according to claim 5, wherein said optically clear material is any of a plastic, a polycarbonate, an optically clear intrinsically antistatic material, a moulded glass or a nylon.

7. An optical detection system according to any of claims 1 to 6, wherein said first optical detector furthermore comprises at least one light source (212) and at least one optical sensor (218)..

8. An optical detection system according to claim 7, wherein said at least one light source (212), said at least one optical sensor (218) and said light guiding device (230) are integrated on a single electronic interconnection substrate (370).

9. An optical detection system (230) according to any of claims 7 or 8, wherein said first optical detector allows measurement of a property of said elongate material (110), said property being at least one of diameter or dimension, presence, movement, colour, transmissivity, absorbance, reflectivity, a surface property, presence of a void or presence of a foreign material.

10. An optical detection system (230) according to any of claims 7 to 9 wherein said at least one light source (212), said at least one optical sensor (218) and said light guiding device (230) are positioned on calibrated positions of the electronic interconnection substrate (370).

11. An optical detection system (230) according to any of claims 8 to 10, wherein said electronic interconnection substrate (370) is a printed circuit board.

12. An optical detection system according to any of claims 7 to 11, wherein said first optical detector comprises a second optical sensor (510) for determining a reference signal from said light source (212).

13. An optical detection system according to any of claims 8 to 12, wherein said at least one optical sensor (218) is calibrated on said single electronic interconnection substrate (370) so as to allow measurements in an absolute way.

14. An optical detection system according to any of claims 7 to 13, wherein the detection system comprises at least one further detector having a sensor (218) for measuring a property of said elongate material (110), said property being at least one of diameter or dimension, presence, movement, colour, transmissivity, absorbance, reflectivity, a surface property, presence of a void or presence of a foreign material.

15. An optical detection system according to claim 13 in combination with claim 14, said sensor of said further detector being integrated on said single interconnection substrate (370).

16. An optical detection system according to any of claims 14 or 15, wherein said first optical detector and said at least one further detector probe the elongate material (110) in the same measurement volume (304).

17. An optical detection system according to any of claims 14 to 16, wherein the detection system furthermore comprises a feedback system between said first optical detector and said at least one further detector.

18. An optical detection system according to any of claims 8 to 17, wherein said detection system furthermore comprises a velocity detector for measuring the speed of a moving material, said velocity detector being integrated on said single interconnection substrate (370).

19. An optical detection system according to any of claims 9 to 18, wherein said convex lens (350) is shaped such that said property can be determined substantially independent of the exact position of the elongate material in the measurement volume (304).

20. An optical detection system according to any of claims 7 to 19, wherein a protection means is provided to seal the optical detection system from dust and/or scratches or to shield for electrostatic, magnetic and/or electromagnetic fields.

21. An optical detection system according to claim 20, wherein the protection means (380) comprises glass plates in the measurement volume.

22. An optical detection system according to any of claims 1 to 21, wherein the measurement volume is provided with a means for making the measurement volume conductive and/or anti-static.

23. A method of manufacturing a detection system for monitoring elongate material, said method comprising
- providing an electronic interconnection substrate (370)
- integrating a light source (212) and at least one light detector (218) on said interconnection substrate (370)
- positioning an integrally moulded piece onto the interconnection substrate to obtain optical components (230) for the detection system.

24. A method of manufacturing according to claim 23, wherein said method furthermore comprises providing protection means (380) for sealing said detection system from dust or for shielding for electrostatic, magnetic and/or electromagnetic fields.

25. A light guiding device (230) for guiding an illumination beam in a region adapted for receiving elongate material to be measured, wherein said light guiding device (230) is a moulded element comprising a means for imaging or conveying the illumination beam in said region, the means for imaging or conveying comprising at least one integrally moulded-in convex lens (350).
